(19) **Europäisches Patentamt / European Patent Office / Office européen des brevets**

(11) **EP 3 056 153 A2**

(12) # EUROPEAN PATENT APPLICATION

(43) Date of publication:
**17.08.2016 Bulletin 2016/33**

(21) Application number: **16153007.6**

(22) Date of filing: **15.11.2013**

(51) Int Cl.:
**A61B 8/08** (2006.01)   **A61B 8/00** (2006.01)
**G06T 7/20** (2006.01)   **A61B 8/02** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.11.2012 GB 201220572**

(62) Document number(s) of the earlier application(s) in accordance with Art. 76 EPC:
**13796123.1 / 2 919 656**

(71) Applicant: **Imperial Innovations Limited**
**London, Greater London SW7 2PG (GB)**

(72) Inventors:
• **FRANCIS, Darrel**
  **Harrow, Greater London HA3 0DZ (GB)**

• **WILLSON, Keith**
  **Echingham, East Sussex TN19 7DB (GB)**
• **DHUTIA, Niti Mahesh**
  **London, Greater London SW7 4AP (GB)**
• **ZOLGHARNI, Massoud**
  **West Drayton, Greater London UB7 7ST (GB)**
• **COLE, Graham**
  **St. Neots, Cambridgeshire PE19 6AT (GB)**

(74) Representative: **Cundy, Jack William**
**HGF Limited**
**140 London Wall**
**London EC2Y 5DN (GB)**

Remarks:
This application was filed on 27.01.2016 as a divisional application to the application mentioned under INID code 62.

(54) **ECHOCARDIOGRAPHY**

(57)   A method for automatically processing an ultrasound image for echocardiography, the method comprising: determining an average velocity vector for tissue movement in each frame of the image; monitoring the change in at least a component of the average velocity vector between subsequent frames to identify at least one of a minimum and a maximum of the (component of the) average velocity vector; and on the basis of the identified maximum or minimum providing cardiac timing information.

Fig. 1

## Description

This invention relates to a method for automatically processing an ultrasound image for echocardiography and also an ultrasound probe which may be used in the method.

## BACKGROUND

There are several clinical problems whose management could potentially be greatly assisted using ultrasound technology all of which is currently available, but not in a form usable by health care professionals at the front line. The necessary technology elements are currently only presented with an intimidating and complex package of cardiac ultrasound (echocardiography) which requires highly-skilled experts to operate. Specialists performing the scans require a great degree of training to distinguish between the subtleties of different abnormalities and grade them accurately.

The scarcity of echo specialists makes it difficult to meet the continuously-rising demand for echocardiography, leading to long waiting lists, even though ultimately only a small minority of such patients require the advanced skills of the specialist. Notably, many scans performed are to assess the function of the heart and turn out to be normal. It would be desirable to provide a system that is designed so that general medical professionals, such as doctors, nurses and other emergency workers at the point of care, who are not trained in echocardiography, can make reliable measurements of specific aspects of cardiac function without the disruptive requirement to bring a specialist to the patient.

Twenty years ago, the availability of an expensive echo machine (£100k) was the rate-limiting step in the wider adoption of cardiac ultrasound, equivalent to three times the annual salary of an echo-specialist. Hardware is now available at less than £10k and the salaries of specialists have become the rate-limiting step; as newer machines become widely available, the situation will become worse and the paucity of trained specialists will be the overwhelming limitation. Since 1997, while the hardware cost of echocardiography has plummeted, the salary of specialists to perform the scan has risen by 80%. It is increasingly illogical to rely on trained specialists to obtain basic echocardiography measurements needed for general, non-cardiological reasons.

In principle, less well-trained staff could, in situations where complex disease was unlikely, acquire images of adequate quality to make important measurements so that a fully-trained specialist is not required. This would allow targeting of this precious resource to the patients likely to have pathology requiring expert assessment.

The number of skilled operators in the UK is only 3,000-4,000. Out of over 600,000 healthcare professionals working within the NHS, less than 1% currently consider themselves able to make basic life-saving measurements, yet almost all of them could do so with a little technological support. Lack of support for beginners in echocardiography arises from the voracious demand for trained specialists who do not have time to train others, and the convention that only specialists, with advanced training, may be trusted to make a complete diagnosis. This latter obstacle is an anomaly for echocardiography, since non-specialists already interpret other tests in which they are not fully expert, such as chest radiographs and electrocardiograms. Furthermore, the opportunity is not limited to hospitals: there are 30,000 doctors in general practice in 10,000 practices in the UK who might benefit from the ability to unequivocally and safely measure the function of their patient's heart during their consultation without having to wait for an outpatient appointment.

## BRIEF SUMMARY OF THE DISCLOSURE

Viewed from a first aspect, the present invention provides a method for automatically processing an ultrasound image for echocardiography. The method comprises determining an average velocity vector for tissue movement in the image, comparing the average velocity vector to a central longitudinal direction of the image, and, on the basis of the comparison, providing an indication of the mis-alignment of an ultrasound probe generating the image.

Thus according to this aspect of the invention, mis-alignment of the ultrasound probe can be determined simply by an analysis of velocity vectors from the ultrasound image (which typically comprises a sequence of frames). This allows the image processing system to provide feedback to the unskilled operator to correct the mis-alignment.

The comparison may be a comparison of the intercept of the average velocity vector with the upper edge of the image and the indication may be an indication of lateral/medial mis-alignment of the probe. In a desired ultrasound image, the apex of the heart should be located at the top centre of the image. If the intercept of the average velocity vector does not intercept the top centre of the image, translational movement of the probe is required to correct the misalignment.

The comparison may be a comparison of the angle of the average velocity vector to the central longitudinal direction of the image and the indication may be an indication of tilting of the probe. Tilting of the probe will cause the average velocity vector to run at an acute angle to the central longitudinal direction of the image.

The method may further comprise processing a further ultrasound image in accordance with the method, the further ultrasound image having been generated in an orientation substantially orthogonal to the first ultrasound image by the same probe, whereby to provide an indication of the mis-alignment of the ultrasound probe in three dimensions.

Viewed from a further aspect the invention provides a method for automatically processing an ultrasound image for echocardiography, the method comprising determining at least one velocity vector for tissue movement at the apex of the image, and if the velocity vector is substantially non-zero, providing an indication of the mis-location of an ultrasound probe generating the image. In a desirable ultrasound image, the apex of the heart, which does not move with the cardiac cycle, should be located at the top centre of the image.

Viewed from a further aspect the invention provides a method for automatically processing an ultrasound image for echocardiography, the method comprising determining an average velocity vector for tissue movement in each frame of the image, monitoring the change in at least a component of the average velocity vector between subsequent frames to identify at least one of a minimum and a maximum of the (component of the) average velocity vector, and on the basis of the identified maximum or minimum providing cardiac timing information.

Thus, according to this aspect of the invention, cardiac timing information can be derived directly from the ultrasound image.

The component of the average velocity vector may be a component in the direction of the apex of the heart in the image. This direction is usually the vertical direction of the image. The monitoring step may comprise at least one of identifying a minimum corresponding to the early diastolic phase of the cardiac cycle, when tissue is moving away from the apex, identifying a minimum corresponding to the late diastolic phase of the cardiac cycle, when tissue is moving away from the apex, and identifying a maximum corresponding to the systolic phase of the cardiac cycle, when tissue is moving towards the apex.

Viewed from a further aspect the invention provides a method for automatically processing an ultrasound image for echocardiography, the method comprising determining velocity vectors for tissue movement in the image, identifying at least a first region of the image in which the average velocity vector is in the direction of the apex of the heart during the diastolic phase of the cardiac cycle, and providing an indication that the first region corresponds to the mitral or tricuspid valve.

Thus, according to this aspect of the invention, the location of the mitral or tricuspid valve can be identified automatically in the ultrasound image. The diastolic phase of the cardiac cycle may be identified by the method described above.

Viewed from a further aspect the invention provides a method for automatically processing an ultrasound image for echocardiography, the method comprising determining velocity vectors for tissue movement in the image, assigning confidence values to the determined velocity vectors, examining a section of the image in a direction substantially transverse to the apical direction of the image, identifying three regions within the examined section having the highest confidence values and highest magnitude velocity vectors, and providing an indication that the three regions correspond to the medial wall, septum and lateral wall of the heart, respectively.

Thus, according to this aspect of the invention, the location of the medial wall, septum and lateral wall can be identified automatically in the ultrasound image.

The method may further comprise automatically locating the mitral or triscuspid valve by the method described above and automatically locating the medial annulus, septal annulus or lateral annulus by reference to the location of the valve and the location of the medial wall, septum and lateral wall.

In the above methods, the velocity vectors may be determined by block matching pixels in sequential frames of the ultrasound image. The method may comprise assigning a confidence value to each determined velocity vector. The confidence value may be determined on the basis of at least one of contrast of the pixels in the matched block, distribution of correlation values of the matched block relative to neighbouring blocks or correlation between the variation of at least a component of the determined velocity vector and (the corresponding component) of the average velocity vector for the image. The method may comprise using specialised real-time prediction techniques to enhance accuracy of the velocity vector and improve the time-efficiency.

The method may comprise identifying a plurality of regions within the image having confidence values above a minimum value. Such regions are typically the areas of interest in the ultrasound image corresponding to the structure of the heart. The method may comprise calculating an average velocity vector for each region. The method may further comprise calculating an average velocity vector for tissue movement in the image as the average of the average velocity vectors for each region.

The invention extends to computer software which configures data processing apparatus to automatically process an ultrasound image for echocardiography in accordance with any or all of the above methods and to data processing apparatus so configured.

Viewed from a yet further aspect, the invention provides an ultrasound probe in combination with a display for representing the ultrasound images obtained by the probe, wherein the display is mounted on the probe for movement therewith. Preferably, the ultrasound images displayed on the display correspond in orientation to the orientation of the probe, in use. Desirably, the scale of movement of the ultrasound images displayed on the display corresponds to the scale of movement of the probe. The display may be detachably mounted to the probe.

The probe may further comprise a mirror mounted to the probe and arranged to reflect an image of the display to a user. Preferably, the probe generates an ultrasound beam in a first plane and the image of the display reflected by the mirror coincides substantially with the first plane. The mirror may be partially reflective, for example half-silvered. Advantageously, the mirror is an appropriately positioned concave spherical mirror, thereby permitting a life-size projection into the body even with a display screen that is substantially smaller than life-size and therefore convenient.

The probe may be configured to obtain ultrasound images in a first plane and in a second plane that is not parallel to the first plane. The probe may comprise a second display for displaying the ultrasound images from the second plane. The relative orientation of the first and second displays preferably corresponds to the relative orientation of the first and second planes. The first plane is typically substantially orthogonal to the second plane.

The probe may further comprise a second mirror mounted to the probe and arranged to reflect an image of the second display to a user. The probe may generate an ultrasound beam in the second plane and the image of the display reflected by the second mirror desirably coincides substantially with the second plane. The second mirror may be partially reflective.

The first mirror and/or the second mirror may be curved whereby to reflect a magnified image of the display to the user. In this way, a life-size image may be obtained using only a relatively small screen. Typically, the mirror is a concave spherical mirror. However, other curved shapes may be used as appropriate. Similarly, it would be possible for the image to be magnified by one or more lenses, rather than a curved mirror, but this adds weight to the probe and is likely to be more complex.

The ultrasound images displayed on the display may be distorted electronically (rather than optically) to compensate for the optical distortion of the magnified image by the curved mirror. Thus, the ultrasound images may be processed by an image processor or image processing software to display on the (first or second) display an image that appears accurate to the user when distorted by the curved mirror.

Viewed from a further aspect, the invention provides an ultrasound probe, particularly for echocardiography, the probe having a longitudinal direction which is the direction of the ultrasound beam emitted by the probe and an end surface which is transverse to the longitudinal direction, the end surface being bounded by a first side and a second side of the probe, wherein the first side of the probe extends further in the longitudinal direction than the second side whereby to encourage tilting of the probe relative to a plane orthogonal to the longitudinal direction in use. The end surface may extend from the end of the first side to the end of the second side and may be oblique to the longitudinal direction. Alternatively, at least the first side extends beyond the end surface of the probe. The extension of the first side beyond the end surface of the probe may be provided by a detachable member mounted to the probe.

Viewed from a further aspect, the invention provides an ultrasound probe comprising a movement sensor, wherein the ultrasound probe is configured to notify an operator when the probe has been located on a patient for longer than a predetermined time period. The ultrasound probe may be configured to stop collecting data when the probe has been located on a patient for longer than the predetermined time period. Because much of the variability that is considered "between operator" in ultrasound measurement is in fact "between probe position", encouraging the operator to reposition the probe several times during acquisition will encourage that operator to acquire a more fully representative selection of positions, so that the mean of the measurements obtained across all of these positions, will better represent the true state of the patient. This would give the advantage of improved test-retest reproducibility of the measurement.

Viewed from a further aspect, the invention provides a docking station for an ultrasound probe, in particular for echocardiography, for holding the ultrasound probe when not in use, the docking station comprising an anatomical model and a holder for the probe, the holder being configured to locate the probe relative to the anatomical model in the correct position for imaging. The holder may be configured to locate the probe relative to the anatomical model in the correct orientation for imaging. The anatomical model may be provided with an image in the region of the probe, which represents the expected ultrasound image when the probe is located in the correct position for imaging.

The invention extends to an ultrasound scanning system comprising data processing apparatus as described and an ultrasound probe as described above, optionally in combination with the described docking station.

## BRIEF DESCRIPTION OF THE DRAWINGS

Embodiments of the invention are further described hereinafter with reference to the accompanying drawings, in which:

Figure 1 is a schematic representation of an ultrasound probe with a display screen according to an embodiment of the invention;

Figure 2 is a schematic representation of an ultrasound probe with a display screen according to another embodiment of the invention;

Figure 3 is a schematic representation of an ultrasound probe with a display screen according to a yet further embodiment of the invention;

Figure 4 shows an embodiment of the invention having two orthogonal screens;

Figure 5 is a schematic representation of an ultrasound probe with a display screen according to a yet further embodiment of the invention;

Figure 6 is a schematic representation of the image created by the ultrasound probe of Figure 5;

Figure 7 shows an example of the display provided to the user according to an embodiment of the invention;

Figures 8 and 9 illustrate the block matching algorithm according to an embodiment of the invention;

Figures 10 and 11 illustrate the correction of probe misalignment according to an embodiment of the invention;

Figure 12 shows the vertical component of the velocity vectors calculated according to an embodiment of the invention, showing the different phases of the cardiac cycle;

Figure 13 shows typical results from automated peak detection according to an embodiment of the invention;

Figure 14 shows the locations of the mitral valve, septal annulus and lateral annulus identified according to an embodiment of the invention;

Figure 15 illustrates the automatic detection of the septum and the medial and lateral walls according to an embodiment of the invention;

Figure 16 shows a speckle tracking velocity curve for an automatically detected annual septum according to an embodiment of the invention;

Figure 17 shows a speckle tracking velocity curve for the left wall according to an embodiment of the invention; and

Figure 18 shows a greyscale apical four chamber freeze frame showing the positions of peak systolic (S'), early diastolic (E') and late diastolic (A') velocities according to an embodiment of the invention.

## DETAILED DESCRIPTION

In broad terms, the present invention provides, in particular embodiments, a rapid, reliable multi-modality cardiac ultrasound imaging system for specialists and novices in clinical practice. The system can provide support to minimally-trained operators to allow them to make measurements of key physiological indices of the heart with the same accuracy as specialists. The system uses automation and quality checks to remove the obstacles due to the parts of the process of making measurements that are challenging to novice operators and result in unreliable measurements. Current cardiological imaging protocols focus on increasingly advanced measurements obtained by increasingly specialised experts using a full-service scanner to answer specific questions on patients with severe heart disease. In contrast, the disclosed system provides non-cardiologist health carers with the opportunity to obtain information urgently required in medicine, outside cardiology. A significant technological element not provided by any ultrasound hardware to date, because it is not conventionally requested by expert cardiologists, is a means of guiding an inexperienced operator to acquire basic crucial information, even if it does not give a complete diagnosis.

There are also novel applications for the disclosed technology in research and audit. The clinical interpretation of ultrasound images depends upon whether or not the image is of sufficient technical quality and contains the relevant information to answer the clinical question posed. There is no objective measure currently available or used that quantitatively defines the quality of the image and the reliability of the associated information for a particular application in comparison with a commonly agreed reference image set. The increasing use of audit as a clinical governance tool in healthcare would be transformed by the capability to objectively measure the quality of images and assess measurement variability. The disclosed technology provides the safety net of quality assurance that a highly-skilled operator, with the benefit of experience, attempts to undertake themselves acting as their own image quality screen, but the novice is not so capable of. The extracted quality parameters developed in the present system could be used as an objective quality descriptor.

The system disclosed herein comprises two main components. The first is a real-time operator guidance system which assists beginners to position the ultrasound probe with only informal peer-to-peer training. The guidance system means that operators do not need an external training course and may simply be guided by a colleague on the ward, who has no special training themselves beyond such peer-to-peer guidance.

The second component is a real-time quality control and measurement system, which permits any operator, experienced or newcomer with very brief training in probe positioning, to make measurements automatically without having to operate any controls on the system, and to have a quality score provided automatically so that the credibility of the measurement can be quantified objectively without the tendency for some overconfident operators to overestimate quality and some tentative operators to underestimate quality.

Together, the system provides advice to the human operator and instructions to the ultrasound scanner that result in real-time extraction and processing of the acquired images.

The guidance system guides the human operator to move the probe to the best location to obtain the desired heart view.

A first important feature of the guidance system is a docking station, which comprises a real-size human torso with a stand for the ultrasound probe which locates the probe touching the torso at the position and angle the operator will emulate on a patient. The torso has a flat upper surface in the plane of intended imaging, and a representative image of what should be found when the probe is correctly positioned.

A relatively short (30-60s) and simple training demonstration is provided to the operator, explaining how the system works and how to obtain the desired images. The demo covers different steps of the process from the correct way of holding an ultrasound probe to how to position and manoeuvre the probe on the chest. The purpose of this short demo is to help the operator intuitively learn how the movement of the probe affects the image by illustration of a 3D heart model and ultrasound beam cutting through it. Preferably, this demonstration video is displayed in a continuous loop (in the manner of a computer display screensaver) whenever the machine is not in active use, for example when the probe is resting in its holder. Additionally, the operator can select to view this demo before conducting an examination.

According to an embodiment of the invention, the ultrasound scanner is provided with a one-to-one image display to form "local virtual reality" for the operator.

The traditional pictorial appearance of the heart on an echocardiograph places the display far from the probe and locates the display at a fixed point in space, because it is commonly attached to the scanner which is resting on a table or on a floor-standing device. The display is traditionally oriented with the position of the probe at the top of the screen and there is an arbitrary scale of relationship between distance within the body and physical distance on the screen. Newcomers find it difficult to instantly associate changes in position of the probe with changes in appearance of the image. They are disadvantaged by not having developed the experts' mental understanding of these interrelationships. Even worse, because the ability to predict how an image will change with different movements of the probe is subconscious, experts find it almost impossible to verbalise instructions to novices, and tend to teach by example or take the probe from the novice and move it themselves to the correct position. This makes the traditional learning process very slow.

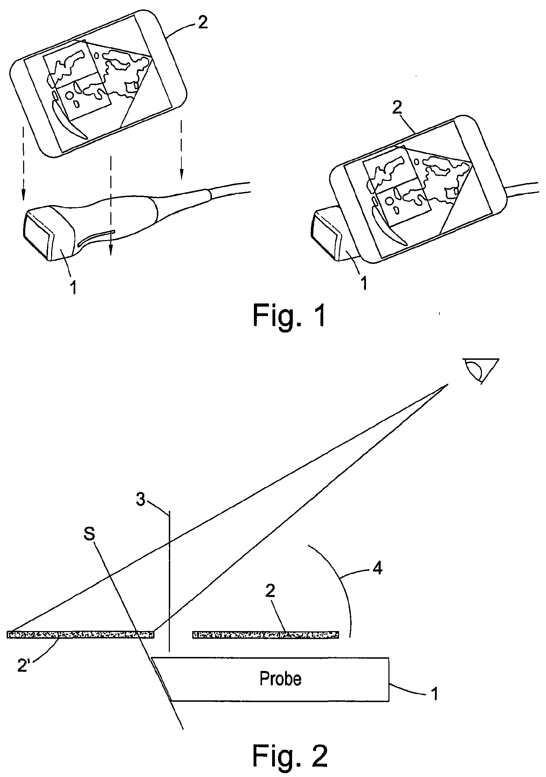

This device according to an embodiment of the invention has four provisions that together substantially improve the ability of newcomers to manipulate the probe position with confidence. Firstly, as shown in Figure 1, a small screen 2 is attached to the ultrasound probe 1. As shown in Figure 1, the screen 2 has size resembling that of a high-resolution digital LCD used on smart-phones, for example around 10cm by 5 cm. The screen 2 is light and moves with the probe 1. Furthermore, the screen 2 is oriented to show the image of the probe pointing in the same direction as the probe 1 is truly pointing in space. Finally, the small screen display is scaled so that one centimetre of movement of tissue corresponds to one centimetre on the small screen 2 itself. The small screen 2 can show not only the live ultrasound images but the display can also be overlaid with guidance information that is calculated in real time (see below).

In the presently preferred embodiment, the small screen 2 is detachable from the probe 1, so that beginners can use it for as many patients as they need to until sufficient experience is gained, or in difficult cases. Users of different levels of experience can detach and re-attach the small screen as required. Attachment devices, well known to those skilled in the art, can be provided to permit such reversible attachment of the small screen to any shape of probe provided as part of commercial ultrasound scanners. In an alternative embodiment, the small screen is permanently fixed to, or part of, the probe.

In a particular embodiment, instead of the user seeing the ultrasound image directly on a small screen 2 located on the probe 1, he sees the image projected into the patient's body in the correct anatomical location. This is achieved in the embodiment shown in Figure 2, by providing the probe 1 with a mirror surface 3 in addition to the display screen 2. Optionally, a shield 4 may be provided. In this embodiment, the display screen 2 is placed on the surface of the probe. The display screen 2 is mounted on the probe 1 at a convenient position back from the probe tip, permitting the user's hand to grasp almost all of the body of the probe 1. The display screen 2 is as described above.

The mirror surface 3 is placed at right angles to, the display screen 2. The mirror surface 3 is a partially or completely reflective surface mounted at an angle that is half way between the angle of the surface of the display screen 2 and the angle of the probe beam within the body. The mirror surface 3 is positioned so that its surface contains the line of commonality between the plane of the probe image and the plane of the display screen 2. These two conditions, on its angle and position respectively, ensure that the projection of the image 2' of the screen 2 for the user to see is in the anatomically correct position inside the body. A completely reflective mirror surface allows maximum clarity of the image; a partially reflective surface, i.e. a half-mirrored or half-silvered surface, permits the user to see both the body surface S and the projected image 2' which may heighten the sense of holographic visualisation of the internal structure of the body.

A shield 4 reduces the opportunity for the user to inadvertently directly view the display screen 2. Such viewing would be undesirable because the display screen's image will appear upside down and will not have the merit of being correctly anatomically located. Advantageously, the shield 4 may be dispensed with if the display screen 2 is selected to be one of the many that has highly directional visibility, oriented so that from the user's perspective, the display is almost illegible, yet from the point of view of the mirror 3, the display is very clear.

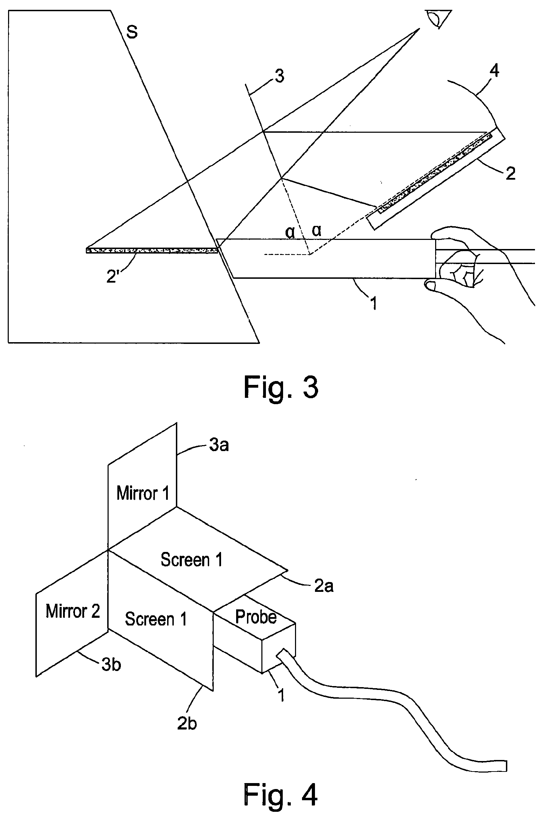

Figure 3 shows a further embodiment of the probe 1 and display 2 according to the invention. In this embodiment, the display screen 2 is pulled back from the tip of the probe 1, and is elevated above it, and tilted forwards. The mirror 3 is placed at the half way angle $\alpha$ between the direction of the probe beam and the orientation of the surface of the display screen 2. The plane of the mirror's reflective surface passes through the line of junction of the plane of the screen surface and the plane of the probe beam.

One major problem for novice operators is that, when they position the probe correctly in one plane, measurements made may not be accurate because they are made obliquely through the heart due to the probe position not being correct in other planes. Whilst experienced operators can recognise when this is happening and reposition the probe, novice operators would have to rotate the probe to a different plane (without changing the position of the probe at all) in order to assess the image in a different plane - a step which is likely to be challenging for all but experienced operators.

Probes already exist which can produce a dual-plane dataset with two orthogonal images. The probe of the invention can optionally use this approach to assist the newcomer to visualise the anatomy in an intuitive way by displaying both of these images in an equally orthogonal display, such that operators can keep the probe position and angle the same, but can move their head to view each display.

The provision of two screens at right angles to each other removes the need for a novice operator to "reconstruct" a 3D shape of the heart, instead focusing on the more achievable task of obtaining adequate probe positioning in each of the two planes.

Figure 4 shows an embodiment of the invention having two orthogonal screens 2a, 2b and two respective orthogonal mirrors 3a, 3b arranged to provide ultrasound images to the user in two planes with each image in the anatomically correct orientation.

Advantageously the dual plane display may be arranged not with the display screens on the surface of (and parallel to) the probe and with the respective mirrors at right angles to the probe, but rather with the display screens pulled back from the probe and at an angle (as in Figure 3), with each mirror at the half-way angle between its corresponding screen and its corresponding probe plane and with the mirror's plane meeting the probe plane at the same line at which the probe plane meets the screen plane, i.e. extending the principle shown in Figure 3 to dual-plane displays. The advantage of this is projection of both images into the anatomically correct position inside the patient, from the point of view of the user.

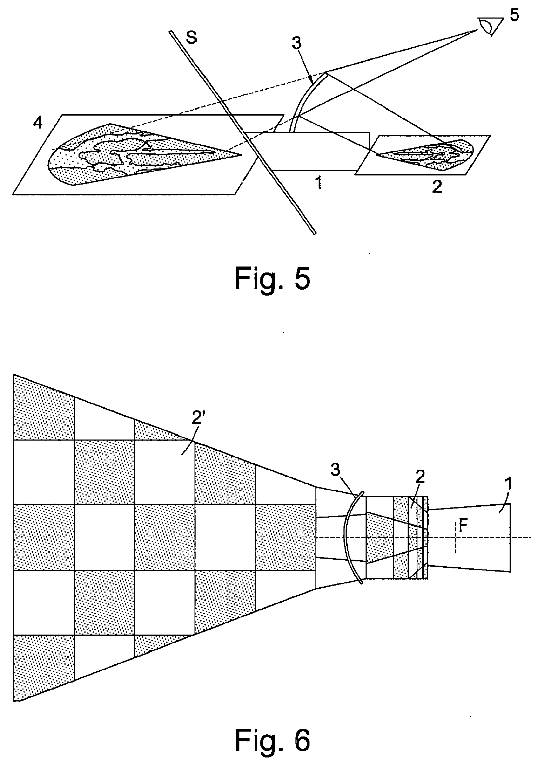

Another embodiment of the invention shown schematically in Figure 5 permits a magnified view of the heart image. In this embodiment, a concave spherical mirror surface 3 is used instead of the flat mirror surface, with the probe 1, small screen 2 and concave mirror 3 positioned relative to the body surface S as shown in Figure 5. The concave mirror 3 provides a life-size projection 2' of the image into the body if the image displayed on the screen 2 is appropriately distorted to counteract the effect of the curvature of the mirror 3. The size of the projection 2' is dependent on the radius of curvature of the mirror, the relative positions of mirror and screen, and the nature of the spatially distorted image displayed on the screen 2.

Preferably the projection 2' is enlarged to life-size, so that each centimetre of movement of the probe 1 on the skin generates a centimetre of movement of the image 2', to maximise the ease with which the user can use common sense to position the probe 1 correctly. However any degree of magnification from the size of the display on the small screen 2 will assist the operator, by enhancing the visibility of the projection 2' without creating the need for a larger and therefore heavier screen.

The dimensions of the spherical mirror 3 and screen 2 are calculated using known optical equations. When a small screen is placed between the mirror 3 and the focal point of the mirror, an observer from the concave side will see a virtual image appearing to arise from the convex side of the mirror 3. This virtual image 2' is upright and magnified. The mirror equation that relates the distance from the mirror to a pixel on the small screen, Ix, the distance from the mirror to the point on the virtual image, Px, and the radius of curvature R of the mirror is:

$$\frac{1}{Ix} + \frac{1}{Px} = \frac{2}{R}$$

where Px has a negative value, and Ix and R have positive values.

The magnification of a mirror is defined as the height of the projected virtual image, Py, divided by the height of the object, Iy:

$$m = \frac{Py}{Iy} = -\frac{Px}{Ix}$$

where Ix is the distance between the mirror and the object and Px is the distance between the mirror and the virtual image. Positions in the x direction that are on the convex side of the mirror have negative values of x, and therefore Px is a negative value.

The image on the screen 2 is displayed in distorted form so that when viewed through the concave mirror 3, its projection 2' is in the correct shape and location. Conveniently this distortion can be carried out algorithmically where for each point (Ix,Iy) on the small screen 2, a corresponding point (Px,Py) on the projected virtual image 2' is identified whose coordinates are computed as:

$$Px = \frac{1}{\frac{2}{R} - \frac{1}{Ix}} \quad , \quad Py = \frac{Px \times Iy}{Ix}$$

The algorithm for display on the small screen is defined as follows. Each physical pixel on the small screen 2 which has controllable colour has a location whose centre may be described as (Ix,Iy) and whose width and height is one pixel by one pixel. For a flat mirror, conceptually, the screen is required to display a colour corresponding to the average tissue characteristic for a rectangle inside the body described by the square whose corners are (Ix-1/2, Iy-1/2), (Ix+1/2, Iy-1/2), (Ix+1/2, Iy+1/2), (Ix-1/2, Iy+1/2). For the spherical mirror considered in this embodiment, the pixel on the screen at (Ix,Iy) must display a colour corresponding to the average tissue characteristic for a quadrilateral shape that is not necessarily a rectangle, because of the distortion induced by the mirror. The quadrilateral shape in the body has the following coordinates for its four corners (denoted by the suffixes_TR: top right, _TL: top left, _BR: bottom right, _BL: bottom left):

$$Px\_TR = 1/(2/R - 1/(Ix+1/2));$$

$$Py\_TR = -Px\_TR * (Iy+1/2) / (Ix+1/2);$$

$$Px\_TL = 1/(2/R - 1/(Ix-1/2));$$

$$Py\_TL = -Px\_TL * (Iy+1/2) / (Ix-1/2);$$

$$Px\_BR = 1/(2/R - 1/(Ix+1/2));$$

$$Py\_BR = -Px\_BR * (Iy-1/2) / (Ix+1/2);$$

$$Px\_BL = 1/(2/R - 1/(Ix-1/2));$$

$$Py\_BL = -Px\_BL * (Iy-1/2) / (Ix-1/2);$$

Conveniently, for each depth of the ultrasound scanner, these coordinates can be calculated for each screen pixel in advance because, apart from the location of the pixel, they depend only on the radius of curvature of the mirror R which is constant. Thus for each pixel, a fixed averaging equation can be pre-defined that encompasses all body positions within the corresponding quadrilateral shape. Conveniently, this process can operate in parallel for different pixels.

By way of example, a small screen, of say 5.5 x 7.5 cm can generate an image with a depth of 27.5 cm ranging in width from 9.4 cm to 30 cm by placing a mirror with a radius of curvature of 20 cm at 2.5 cm from the skin and 2 cm from the closest edge of the screen.

Figure 6 illustrates (using a checkerboard pattern) the manner in which structures inside the body are displayed on the small screen 2. Objects at small depth (near the probe 1) are displayed with only small reduction in size on the small screen 2. Objects at greater depth are displayed with greater reduction in size on the small screen. Any practically required degree of depth can be appropriately represented, because of the progressive reduction in size closer to the focal point F of the curved mirror 3. In principle, even objects at infinite depth can be displayed, if the small screen's extent reaches the focal point (F) of the mirror. While all parts of the image are displayed life-size in the virtual image, the parts that are furthest from the probe have undergone the greatest magnification from their display on the small screen. In other words, the small screen representation of these far parts uses a relatively small number of pixels to display any given physical area (e.g. in square millimetres) within the body. As a consequence of this magnification, it is desirable for the small screen to have a high spatial pixel resolution, i.e. a large number of pixels per unit distance particularly in the direction towards/away from the probe 1. The evolution of the design of high-resolution displays has typically been towards square pixels (or in general the same resolution in two orthogonal directions), and towards an ideal of the highest resolution that is detectable by the unaided eye and typical viewing distance for a cellphone. The current state of the art of this is the iPhone which has square pixels and a resolution of 326 dots per inch in each direction. Where asymmetrical resolutions are available, it is advantageous to orient the higher-resolution axis of the screen so that it points towards/away from the probe. It is also noted that the radial resolution of the current state of the art of ultrasound imaging is much higher than the lateral resolution. For this reason increased screen resolution will readily translate into greater resolution of the clarity in the virtual image in the radial direction especially for parts of the image far from the probe.

We have identified that newcomers to echocardiography characteristically point the probe at an angle that is too orthogonal to the skin surface, even when advised not to. The reason for this appears to be the generic shape of the probe surfaces currently used, which are designed to be orthogonal to its long axis to permit the same probe to be used in many circumstances. In a particular embodiment, the probe is configured to encourage the newcomer to tilt the probe appropriately. As shown in Figures 2 and 3, the probe 1 may be configured to have an end surface that is oblique to the direction of the ultrasound beam. This may be achieved by the configuration of the probe itself. Alternatively, an oblique surface may be added to a standard probe, for example by means of a gel wedge, to allow a desired direction of emitted ultrasound beam to obtained. As a further alternative, an extension may be provided to one side of the probe, which encourages the user to tilt the probe away from the extension. In this case, end surface of the probe is not oblique to the direction of the ultrasound beam, but the angle of incidence of the ultrasound beam on the skin is still non-zero. For example, a planar flange, preferably part of the small screen attachment device, is added near the tip of the probe that obscures the probe tip and makes visible only a surface whose orientation seeks to passively guide the user to favour the typically correct orientation.

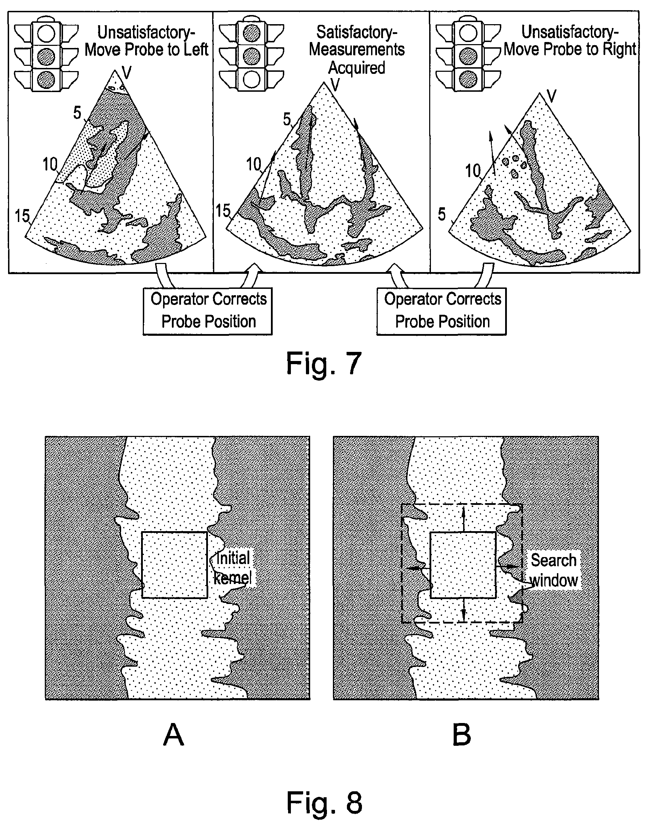

According to an embodiment of the invention, the real-time scanning system automatically analyses the obtained image data in real time and provides the operator with simple guidance via the display on how to make adjustments to the probe position to acquire images of quality suitable for interpretation and analysis. Figure 7 shows an example of the display provided to the user arrows show the direction of heart muscle movements to be automatically obtained from consecutive frames.

In an embodiment of the invention, block matching methods are used to track myocardial tissue motion on the envelope-detected B-mode grey-scale images. Such methods are known for example from: L.N. Bohs, G.E. Trahey, "A novel method for angle independent ultrasonic imaging of blood flow and tissue motion", IEEE Trans Biomed Eng, 38 (1991), pp. 280-286; L.N. Bohs, B. Geiman, M. Anderson, S. Gebhart, G.E. Trahey, "Speckle tracking for multidimensional flow estimation", Ultrasonics, 38 (2000), pp. 369-375; and Goffinet, C. & Vanoverschelde, JL. (2007), "Speckle Tracking echocardiography", In: European Cardiovascular Disease.

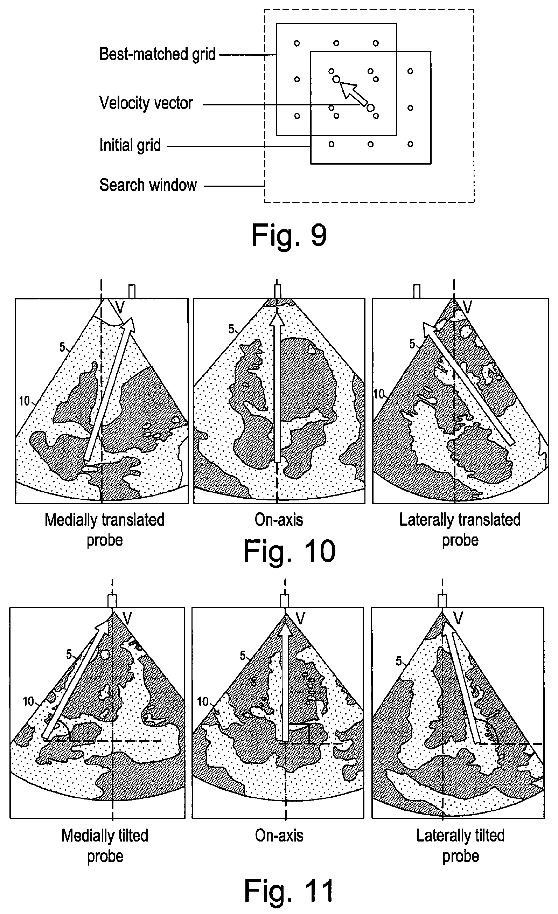

A similarity measure is computed between the intensity pattern of a grid of pixels of dimensions m×n (kernel) in one frame, and a set of identically sized kernels in the next frame (see Figures 8 and 9), to find the best matched kernel using one of the following similarity measures:

- Sum of squared differences where A and B are the grid patterns and m and n are the dimensions of the kernel.

$$\sum_m \sum_n (A_{mn} - B_{mn})^2$$

- Sum of absolute differences where A and B are the grid patterns and m and n are the dimensions of the kernel.

$$\sum_m \sum_n |A_{mn} - B_{mn}|$$

- Correlation using the Pearson product-moment correlation coefficient, r, where A and B are the grid patterns, $A_{mean}$ and $B_{mean}$ are the means of A and B respectively, and m and n are the dimensions of the kernel.

$$r = \frac{\sum_m \sum_n (A_{mn} - A_{mean})(B_{mn} - B_{mean})}{\sqrt{\left(\sum_m \sum_n (A_{mn} - A_{mean})^2\right)\left(\sum_m \sum_n (B_{mn} - B_{mean})^2\right)}}$$

The presently preferred embodiment uses the sum of squared differences similarity measure. The dimensions can be optimised through trial and error and a-priori knowledge of the image properties.

In Figure 8 the position of the initial kernel of pixels for block matching in the first frame A of a 2D greyscale image is shown. The second frame B shows an additional boundary of the search window (white) for shifted kernels. The arrows illustrate how the kernel is shifted in all combinations of directions, but only within the boundaries of the search window. Figure 9 shows the initial kernel (dark square) with all the shifted kernels within the search window (grey dotted line) yielding the best-matched kernel (light square) and a velocity vector (arrow) constructed representing the displacement of that section of tissue from one frame to the next.

The kernels with which the initial kernel is compared are constructed by shifting the initial kernel in all the directions, preferably in one pixel steps, within a specified search window in the next frame. The lowest sum of squared differences between the initial kernel and the shifted kernels, gives an indication of the most probable direction of movement of the tissue. A velocity vector is then constructed by measuring the displacement from the initial kernel to the best-matched shifted kernel. This process is repeated across the entire image, and for each pair of consecutive frames. The output from the speckle tracking algorithm is a dynamic velocity vector field across the entire image. Each velocity vector represents the direction and distance moved by each segment of tissue on the image from one frame to the next. Advantageously, and provided enough processing resources, for tracking the tissue motion, rich RF data, including amplitude and phase, can be used that provides a more precise velocity vector field. The concept of tracking is similar to the B-mode tracking outlined above.

To assist in time-efficiency of this system to operate in real time, an embodiment of the invention uses a course-to-fine block matching approach, i.e. progressing from coarse to fine kernel sizes to reduce processing power required. Such a method is known, for example from F. Yeung, S.F. Levinson, K.J. Parker. "Multilevel and motion model-based ultrasonic speckle tracking algorithm", Ultrasound Med Biol, 24 (3) (1998), pp. 427-441.

Advantageously, to assist in time-efficiency and accuracy of the dynamic velocity vector field, an embodiment of the invention uses estimation techniques such as Kalman filtering or other similar known techniques. This can be achieved by accumulating a record of the dynamic velocity vector field obtained over a series of frames, and incorporating this data as a-priori information into the block-matching process for the next frame. This helps by narrowing the search window, which is beneficial in terms of both searching time and accuracy. Conveniently, the velocity data from frames spanning a period such as 100ms is fitted to a polynomial or other smooth function of time by known methods, and this used to extrapolate the most likely velocity for the next frame. When the image of the next frame is available, it is then most intensively studied for potential velocities near the predicted velocity. This allows the computing power of the system to be preferentially dedicated to the range of potential velocities which is most likely to be the actual velocity and thereby allows any given level of hardware power to give the most accurate results.

The block matching algorithm locates the best matched kernel by minimising a cost function as described above, and therefore estimates the velocity to the nearest pixel. In order to improve the precision of tissue tracking to track motion to the nearest 0.1 pixels, sub-pixel tracking is used, for example parabolic fitting and grid slopes, as described in B. Geiman, L. Bohs, M. Anderson, S. Breit, and G. Trahey, "A comparison of algorithms for tracking sub-pixel speckle motion." in Ultrasonics Symp. Volume 2: IEEE, 5-8 ct 1997, pp. 1239-1242 or Zahiri-Azar, R., Goksel, O., Yao, T.S., Dehghan, E., Yan, J., and Salcudean, S.E., "Methods for the estimation of sub-sample motion of digitized ultrasound echo signals in two dimensions," EMBS 2008. 30th Annual International Conference of the IEEE 5581-5584 (2008).

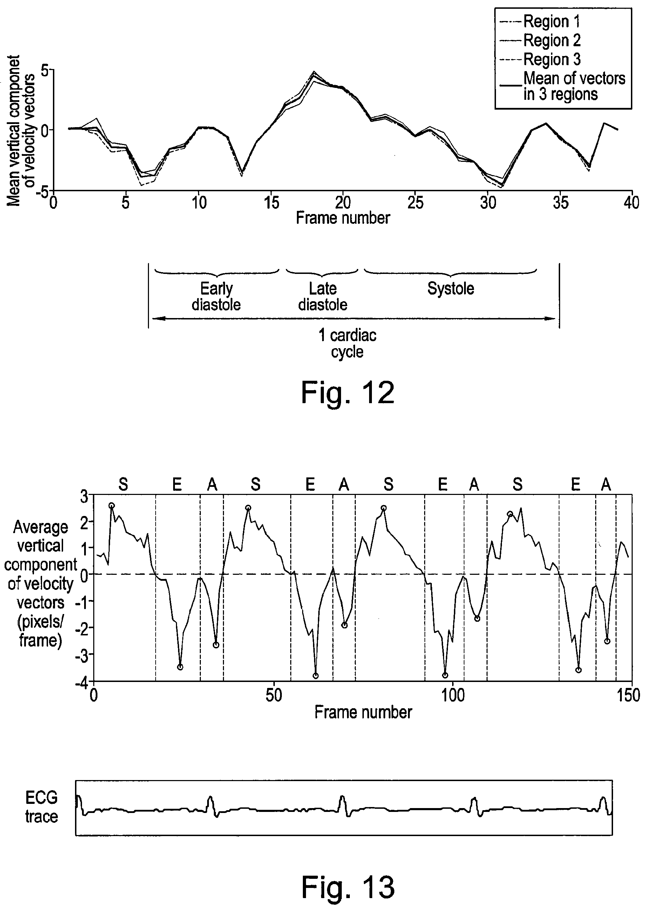

The preferred approach uses a parabolic fitting method, where two orthogonal parabolic curves are fitted to the horizontal and vertical sum-of-squared-differences (SSD) error values along the optimum position, and the minimum for each of the fitted curves is located. This makes it possible to estimate the horizontal and vertical components of the velocity vector to a precision of even less than 1 pixel, for example as low as 0.1 pixels. Based on the parabolic model shown in Figure 12, the following equation of a parabola is used to locate the horizontal position (the vertical position is located similarly):

$$y = ax^2 + bx + c$$

The true minimum of the curve is found by differentiating and setting the derivative to zero.

$$\frac{dy}{dx} = 2ax + b \qquad\qquad x = -\frac{b}{2a}$$

Substituting the pixel values for each of the three known data points into the equation above gives:

$$s_1 = a - b + c$$
$$s_2 = c$$
$$s_3 = a + b + c$$

The lateral displacement/shift is given by the parameter $x_0$. Therefore, to calculate the sub-pixel shift, the following formula is used:

$$x_0 = \frac{s_1 - s_3}{2s_1 - 4s_2 + 2s_3}$$

where $s_2$ is the minimum SSD error term from the grid, and $s_1$ and $s_3$ are SSD terms from adjacent positions on either side.

This ultrasound imaging system according to an embodiment of the invention can operate without the human operator indicating regions of interest. The device automatically defines a degree of confidence to distinguish between noisy vectors and reliable vectors that show valid tissue movement. In order to do this, a combination of global image characteristics, block-matching characteristics and velocity vector characteristics, some of which are known per se are used to assign a confidence level score to each velocity vector, indicating its validity. Our preferred embodiment uses all of the following characteristics to optimise the speckle tracking output. However, any combination of these characteristics could be used.

The contrast of each kernel from the block matching is used as a reliability measure of the velocity vectors, because it is expected that areas with low contrast will be the black regions within the chambers of the heart and the bright white noisy regions, whereas the regions of interest, i.e. the myocardial tissue have a relatively high level of speckle and therefore a very high contrast. The contrast of each region of tissue is determined by applying one of the many methods available, preferably the Michelson contrast formula to each mxn kernel defined in the block matching stage. The Michelson contrast, CM, is defined by:

$$C_M = \frac{I_{max} - I_{min}}{I_{max} + I_{min}}, \qquad 0 \le C_M \le 1 \quad since \quad I \ge 0$$

where $I_{max}$ and $I_{min}$ are the maximum and minimum luminance within the kernel (see Michelson, A. (1927). Studies in Optics. U. of Chicago Press).

Each kernel's contrast value is rescaled from 0 to 1 by dividing by the maximum contrast value. The regions of interest are classified as those with a contrast value greater than 0.1, and the higher the contrast value the greater the confidence value of the velocity vector in that region as it contains relatively more speckle information than the lower contrast regions. Conveniently, this reduces the speckle tracking processing time since regions with a low contrast do not need to be processed during block matching.

During block matching, each initial kernel is compared with its neighbouring kernels, resulting in a grid of sum of squared differences (SSD), with one minimum i.e. the optimum position indicating the most probable direction of movement of the tissue. Conveniently, the distribution pattern of values in this grid can be used to determine the reliability of each vector, based on the assumptions that:

    i. The value of the SSD progressively increases at positions progressively further either side of the optimum;
    ii. The SSD values along the vertical and horizontal directions of the optimum position have a parabolic shape;
    iii. If the pattern is not a single parabolic curve, with a distinct minimum, it is an unreliable vector.

Automatic detection of whether the pattern is a parabolic curve can be achieved by many methods including conveniently the well-known $R^2$ value.

Advantageously, this property can be used to compute the percentage of velocities at the extremes of the search window, in order to evaluate the parameter settings for block matching. For instance, if the majority of velocities lie within the extremes for a particular image sequence, this would indicate that the block matching search window is not large enough to correctly capture the myocardial motion for this image sequence.

Skew is a statistical measure used to test the asymmetry of a distribution. In the particular embodiment, the skew is used to observe the distribution of the sum of squared differences (SSD) between an initial kernel and all the shifted kernels. The skew is calculated as the ratio of the 3rd moment about the mean to the standard deviation (the 2nd moment).

If the distribution has a longer left tail, it is known as negative skew, and the opposite is true for positive skew. If both left and right tails are equal then the skew is zero, as would occur with a normal distribution.

To construct the velocity vector, the lowest SSD value is normally selected to indicate the most probable direction of movement. However this will give invalid results if all or most of the SSD values for a kernel have similar values, i.e. in a noisy region of the image, as the algorithm will typically select the lowest of these SSD values to be the direction of movement. To prevent this, the skew is used to ensure that the kernel selected has a significantly lower SSD value than the rest of the shifted kernels, i.e. the distribution is negatively skewed. A negative skew in this case will show that the majority of SSD values are much higher than the selected minimum SSD.

There are a number of neighbourhood consistency quantification methods used in speckle tracking (see for example Thomas H. Marwick, Cheuk-Man Yu, Jing Ping Sun. (2008). "Myocardial Imaging: Tissue Doppler and Speckle Tracking", Chapter 2. John Wiley & Sons), which are used to validate the vectors. In the particular embodiment, each speckle-tracked velocity vector is compared with its four neighbouring vectors and assigned a score, which is the cosine of the angle between the velocity vector, and its neighbouring vector. Comparison of each velocity vector with its neighbour gives a value from -1 to 1, with -1 indicating vectors at 180° to each other, and 1 indicating no difference in direction between the two vectors. Each vector is compared with its four neighbouring vectors, and the scores are added together to give an overall score ranging from -4 (lowest consistency) to 4 (highest consistency).

The temporal consistency of each vector can be investigated by comparing each velocity vector's vertical component across time with the average vertical component velocity vector trace (average of the vertical components of all the velocity vectors in each frame), using one of the similarity measures well known to those skilled in the art, preferably the Pearson product-moment correlation coefficient:

$$r = \frac{\sum_{i=1}^{n}(X_i - \bar{X})(Y_i - \bar{Y})}{\sqrt{\sum_{i=1}^{n}(X_i - \bar{X})^2}\sqrt{\sum_{i=1}^{n}(Y_i - \bar{Y})^2}}$$

where X and Y are the two variables being correlated and n is the number of samples.

This characteristic exploits the unique property of typical motion of the heart during the different phases of the cardiac cycle. For instance, during systole the myocardial tissue contracts towards the apex, and during early and late diastole, the movement is away from the apex. Using this property, the velocity profile across time of the velocity vectors within the regions of interest, namely the septum and ventricular walls, would typically follow a specific pattern and correlate strongly with the average velocity trace, whereas velocity vectors in the noisy regions would be expected to have a poor correlation with the average velocity trace.

In the particular embodiment, the novice is guided to acquire the desired view, by using a combination of the following image processing techniques and visual aids:

i. A visual guidance is displayed on the screen, in the form of two or three landing zones or boxes, encouraging the operator to place specific landmarks of the image into these regions. The three landmarks are the septum and the two ventricular walls. In the particular embodiment, two boxes are used, one for the septum and one for the lateral wall.

ii. Spotlight area - The regions of interest in the image are highlighted, for example as green pixels in the greyscale image, to indicate to the operator which regions of the image will be used to make the measurements, encouraging the operator to maximise the areas of highlighted tissue.

iii. Image processing methods, described in the following, are used to determine tilting and translation of the probe and provide guidance to the operator to fine-tune image acquisition.

In a particular embodiment of the invention a series of image processing techniques are used to:

- Determine the orientation of the images in real-time using speckle-tracked vectors

- Relate orientation to probe positioning

- Provide guidance to the operator on how to manoeuvre the probe to fine-tune image acquisition.

Once the reliable velocity vectors are determined using the confidence level characteristics described above, a fully automated region growing method, based on known methods can be implemented to locate the regions of highest velocity, in particular the septum and ventricular walls. The three regions are grown around the pixels with high velocities and high confidence level weighting.

The preferable region growing method consists of the following steps, which are repeated for each of the two or three predefined landing zones described in the previous section, conveniently limited to the two left ventricular walls, i.e. the septum and lateral wall:

a) Select a seed pixel within the predefined region, which is the point with the maximum weighting, i.e. a high confidence level and high velocity vector.

b) Selects a vector from the 8 neighbouring velocity vectors, with a weighting closest to that of the seed pixel. This velocity vector is added to the region, increasing the size of the region.

c) The new region is assigned an average weighting (weighting of all the velocity vectors within the region).

d) The neighbouring velocity vectors to that region are compared to the new average weighting, and the value closest to the average is added to the region.

e) Steps a-d are repeated until the difference between the average weighting of the region and weighting of the surrounding velocity vectors exceeds a specified value.

f) To find the next region, the highest weighted velocity vector from the next predefined region is selected and labelled as the new seed pixel, followed by region growing. Steps a-f are repeated until the two/three regions are found.

g) The final output from the algorithm is the three regions, ideally expected to be the septum and two ventricular walls.

h) Preferably, when searching for regions, very few constraints should be applied to ensure that the device will be able to produce reasonable results for any patient, regardless of the changes in shape or size of the heart.

The image orientation information can be used in the following ways:

In apical 4-chamber images, medial and lateral probe translation affects the position of the apex of the heart in relation to the centre of the image. Since the ventricles normally contract longitudinally towards the apex during systole, the overall mean velocity vector, $V_i$ (computed during speckle-tracking) points towards the apex of the heart:

$$V_i = \frac{1}{r} \sum_{k=1}^{r} v_{ik}$$

where $V_i$ is the overall mean velocity vector for frame i, and $v_{ik}$ is the mean regional velocity of region k for frame i, obtained using the following definition:

$$v_{ik} = \frac{1}{n} \sum_{j=1}^{n} u_{jk}$$

where $v_{ik}$ is the mean regional velocity of region k in frame i, n is the number of vectors within the region of interest, and $u_{jk}$ is the velocity of the vector j, which lies in the region k.

By locating the position of the apex using this mean velocity vector, it is possible to identify whether the apex is horizontally centred in the image. Any displacement of the average velocity vector to the left or right of the centre of the image corresponds to medial or lateral translation of the probe from the desired on-axis view.

Using the cardiac timing curve (described below) to identify the systolic and diastolic phases of the cardiac cycle and averaging the overall velocity vectors using only those frames, it is possible to obtain an average orientation vector for the image.

The intercept of the resulting orientation vector with the top edge of the image indicates the position of the apex of the heart in that image. The algorithm calculates the displacement of this intercept to the left or right, relative to the centre of the image. The displacement is measured in pixels and then converted to cm using the known image scale.

Figure 10 illustrates how the intercept of the overall average velocity vector can be used to detect the direction and amount of probe translation. In Figure 8, 2D greyscale apical 4-chamber images show how the intercept of the overall mean velocity vector (arrow) with the top of the image, relative to the centre (dashed line), can be used to distinguish between an on-axis (middle) and medially (left) or laterally (right) translated probe positions. In the particular embodiment, this information is fed back to the operator in the form of an arrow indicating the direction in which the probe needs to be moved.

The orientation of the image can be expressed in the form of an angle, and this information can be used to determine misalignment of the image due to medial/lateral tilting of the ultrasound probe. Figure 11 shows 2D greyscale apical 4-chamber images showing how the orientation angle of the overall mean velocity vector (arrow) with the top of the image can be used to distinguish between on-axis (middle) and medially (left) and laterally (right) tilted probe positions. The orientation angle is calculated by averaging the horizontal and vertical components of the speckle-tracked velocity vectors to get an overall vector of tissue motion for each region, for each frame, $v_{ik}$, as described above.

The average regional vectors are averaged to get one overall velocity vector for each frame, $V_i$, in Cartesian coordinates. The velocity vector is then converted from Cartesian coordinates to polar form to obtain an orientation angle, taking into account the relevant quadrants. The final orientation angle is calculated by averaging the orientation angles during the systolic phase using only systolic frames. The resulting orientation angle is defined as follows: an angle less than 90° indicates a medially tilted probe, and greater than 90° indicates lateral tilting of the probe. An on-axis image is in the range of $90 \pm 5°$.

By using acquisition of images in two orthogonal planes, the orthogonal plane to a 4-chamber view should display a 2-chamber view, and therefore has similar tissue motion characteristics. By extending and applying the image processing analysis, described above, the device can provide tilting/translational probe guidance in the superior/inferior plane of motion.

Apical tissue is expected to be stationary, while the myocardial tissue contracts towards and away from it. If the ultrasound plane cuts the heart obliquely, the velocities at the apex in the image will not be zero, since this is not the 'true' apex. Therefore, the device can distinguish between on and off-axis images by investigating the profile of the speckle-tracked velocities along the septum. For an on-axis image, velocities should decrease to zero towards the apex.

Determination of image quality (see: Wang Z. A, "Universal Image Quality Index", IEEE Signal Processing Letters. 2002;9(3):81-4; Huitao L., "A training-based no-reference image quality assessment algorithm", International Conference on Image Processing, ICIP '04; 2004; and Xin L., "Blind Image Quality Assessment", IEEE International Conference on Image Processing, 2002;1:449-52) is an important component of the described system as it can:

i. Prevent novices from unknowingly using sub-optimal images to make measurements;

ii. Provide an independent confidence measure of the performance of the system based on the quality of the image obtained; and

iii. Estimate the end-point of an acquisition once an adequate image is obtained and measurements are made with satisfactory confidence intervals.

A unique property of myocardial tissue is its consistent temporal pattern of contraction. The velocity of the tissue follows a distinctive profile, with the systolic phase, followed by the early diastolic and late diastolic phases. All the tissues in the heart normally contract following the same pattern. Therefore, this property is useful as a measure of image quality.

In a particular embodiment, this temporal consistency is investigated by computing the Pearson product-moment correlation coefficient of each block matching kernel's velocity profile across time with the average velocity profile across time of the regions that potentially contain myocardial tissue. The Pearson product-moment correlation coefficient, r, is defined as:

$$r = \frac{\sum_{m}\sum_{n}(A_{mn} - A_{mean})(B_{mn} - B_{mean})}{\sqrt{\left(\sum_{m}\sum_{n}(A_{mn} - A_{mean})^2\right)\left(\sum_{m}\sum_{n}(B_{mn} - B_{mean})^2\right)}}$$

The percentage of these kernels, $P_{valid}$, that have a strong correlation (preferably r = 0.6) between their individual and the average velocity profile can be used as a measure of the image quality index. A good quality image will have relatively more regions of interest with clearly defined myocardial tissue which will result in a high percentage of kernels correlating with the average velocity profile. On the other hand, a poor quality image will have a high percentage of kernels consisting of noisy regions, and the velocity profiles of these kernels will be dominated by noise, and therefore will not follow the characteristic pattern of the cardiac cycle. The percentage of kernels which follow the characteristic velocity profile is a useful indicator of the proportion of the image with useful information, and hence an indicator of image quality.

Echocardiography scans usually involve recording the timing of the cardiac cycle simultaneously, using electrocardiogram (ECG) leads. The ECG provides useful information on the different phases of the cardiac cycle. A number of echocardiography measurements rely on an echocardiographer manually identifying time points of specific events in the cardiac cycle, using the ECG.

For an operator guidance system, it is useful for the system to be able to identify the different phases of the cardiac cycle automatically, using the ultrasound data being acquired, without requiring that the operator put ECG leads in place, since several of the guidance algorithms rely on cardiac timing information. This is especially helpful in situations where the ECG may not be clinically convenient, such as in emergency cases, or when a hand-held ultrasound scanner is used to test a series of patients without introducing the delay inherent in connecting and disconnecting ECG cables from the individual patients. Previous methods to detect cardiac timing include using B-mode intensities and colour tissue manifold learning (see Aase SA, Snare SR, Dalen H, Støylen A, Orderud F, Torp H, "Echocardiography without electrocardiogram", Eur J Echocardiogr 2011;12:3-10.; and Gifani P, Behnam H, Shalbaf A, Sani ZA, "Automatic detection of end-diastole and end-systole from echocardiography images using manifold learning", Physiol Meas 2010;31:1091-103).

An embodiment of the present invention uses speckle tracking to detect the phases of the cardiac cycle without an ECG, according to the following method:

1. The velocity vectors in each region of interest k are averaged across space to obtain a mean regional velocity curve across time, $v_{ik}$, where $v_{ik}$ is the mean regional velocity of region k in frame i.

2. The mean regional velocity vectors can be averaged to obtain an overall mean velocity vector, $V_i$, for each frame, where $V_i$ is the overall mean velocity vector for frame i.

3. A plot of the vertical component of the mean (regional or overall) velocity vectors clearly indicates cardiac timing information; in particular the systolic and diastolic phases of the cardiac cycle and the frames in which they occur. Figure 12 shows the vertical component of the velocity vectors from each region, found from the automated region growing algorithm, showing the different phases of the cardiac cycle. The upward peak (positive velocities) represents the systolic phase of the cardiac cycle, and the two downward peaks (negative velocities) represent the early and late diastolic phases.

4. The systolic and diastolic segments can be automatically determined using one of the many known peak detection methods available, such as derivative-based algorithms, or non-linear filtering algorithms. The particular embodiment implements the following steps:

    a. Apply a low-pass, for example moving average, filter to smooth the curve and remove high-frequency noise;

    b. Locate local maxima by searching for points on the curve preceded by values lower than a specified delta value.

    c. Locate local minima by searching for points on the curve preceded by values greater than a specified delta value.

Figure 13 shows typical results from the automated peak detection, in which automated peak detection is circled and the segmentation of the cardiac cycle curve into systolic (S), early diastolic (E) and late diastolic (A) phases is represented with dashed lines. The corresponding ECG trace, acquired simultaneously is displayed below the velocity curve.

The process of block matching for speckle tracking can be computationally very expensive. However, in order for the system to run in real time, speckle tracking must be accelerated to meet the real-time constraints. To this end, a preferred arrangement uses parallel processing whereby multiple kernels can be processed simultaneously. In our particular embodiment, parallel execution of the speckle tracking algorithms is carried out using the computing power of the ultrasound scanner. This allows the present system to run on an ultrasound scanner without the need for an extra processing unit. However, if the processing power of the ultrasound scanner is insufficient to perform the speckle tracking in real time, the computationally intensive steps can be carried out on an external processing unit (such as a PC) that is powerful enough to offer the required accelerations. To this end, a communication method may be established between the ultrasound scanner and the external processing unit to extract the acquired images from the scanner in real time to carry out speckle tracking. Network-based communication protocols are suitable for this purpose.

A preferred method of parallel processing is the use of one or more (Compute Unified Device Architecture) CUDA-enabled Graphics Processing Units (GPU) for parallel implementation of the algorithms. Most powerful GPU cards (Tesla) are currently available only on desktop-sized computers. However, expected technological improvements over the next 3-4 years will make such cards available on laptop-sized portable computers which are ideal as the external processing units for the present system.

The system disclosed herein is further capable of making diagnostic measurements automatically, requiring no input from the operator. In general, the system aims to automatically identify the location of optimal measurement sites (within the image plane) and make measurements of blood flow and myocardial movement/function using a combination of any or all of: speckle tracking, colour flow mapping, PW Doppler information, and colour m-mode. The key stages are:

- Identification of physiological landmarks and optimal measurement sites (e.g. mitral inflow, septal annulus, lateral annulus) for automated quantification;

- Automatic instruction to the ultrasound equipment to move the cursor to that point, switch mode to the appropriate Doppler modality, and acquire a strip of Doppler data;

- Immediate processing of the data to yield numerical values together with the quantitative index of confidence;

There are a number of common and fatal clinical problems in which accurate measurements of cardiac parameters by ultrasound would result in a paradigm-shifting advance in care. These are described in the following.

The E (the velocity of blood flow across the mitral valve) and E' (the peak rate at which the ventricle expands to receive this inflow of blood), especially expressed as the E/E' ratio shows promise as a surrogate of left ventricular filling pressure, with both diagnostic and prognostic value, and the S', E' and A' tissue velocities at the mitral annuli provide useful information on left ventricular function. However, selecting the locations to make these measurements currently needs to be done manually by echo specialists, and in the absence of guidelines stating exactly where to measure, these measurements are open to variability. The system disclosed herein uses speckle tracking and colour flow mapping to select Doppler measurement positions at the mitral inflow, septal annulus and lateral annulus, and makes measurements of peak tissue (S', E' and A') and blood (E) velocities using speckle tracking and from the Doppler traces obtained at these positions, without any user input ( see also Dhutia NM, et al (2012), "A new automated system to identify a consistent sampling position to make tissue Doppler and transmitral Doppler measurements of E, E' and E/E'", Int J Cardiol. 155(3):394-9.)

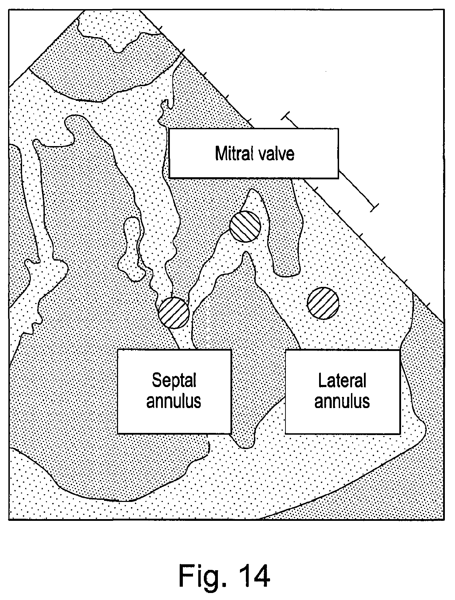

One of the uses of identifying the diastolic phase of the cardiac cycle is to exploit the unique anti-phase behaviour of the mitral and tricuspid valves during this time. When the myocardial muscle relaxes, it moves away from the apex, whereas since this is the time the ventricles are filled, the valves open and hence are the only structures within the image moving towards the apex. Using this information, the regions of velocity vectors pointing apically during the diastolic frames of the video loop, are identified as the mitral and tricuspid valves. An example location of automatically selected mitral valve is shown in Figure 14.

A particular embodiment of the present system uses this information about valve location in several ways:

- To identify the region within which to search for the mitral valve inflow sample volume position.
- To determine the horizontal level of the valve and annulae, and use this to validate sample volume selection at the annulae.
- To use the position of the valves to slightly alter the boxes for probe positioning guidance, to make it easier for the operator to place the landmarks in those boxes.

One of the following methods can be used to locate an approximate initial region for the location of the septum and two ventricular walls:

The operator is instructed to align the image placing the septum and ventricular walls in the designated landing zones/boxes, which therefore can be used as the approximate location of those regions.

The overall confidence level weighting from the speckle tracking stage across time. By averaging each column of the image matrix, a trace is obtained with three peaks at the regions with a high velocity and a high overall confidence level weighting:

$$I_x = \frac{1}{n} \sum_{y=1}^{n} i_{xy}$$

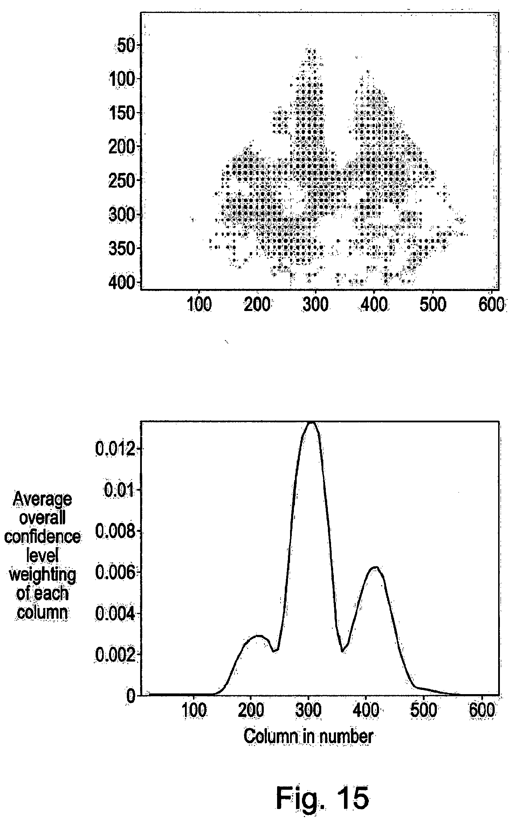

where $I_x$ is the overall confidence level weighting for column x, and $i_{xy}$ is the confidence level weighting at position (x,y). These peaks indicate the location of three regions of interest, namely the septum and the two ventricular walls, along the horizontal axis. Figure 15 shows the combination of overall confidence level weighting across time for an apical four chamber image. The intensity of each dot represents the weighting of the velocity vector in that position. The trace below results from column-wise averaging of the top image. Peaks are observed at the regions with high velocity and high confidence level weighting, expected to be the approximate horizontal locations of the regions of interest, i.e. the septum and the medial and lateral walls.

The horizontal level of the annulae can be determined by using the valve location as described in the previous section. A touch-sensitive screen may be provided to give the option to the operator to select the approximate regions on the screen where they can see the two annulae.

The exact measurement position at the septal and lateral annulus can then be located by searching, for the points with the maximum velocity within each of the approximate regions for each frame (see Figure 14).

Additionally, in order to prevent significant changes of the sample volume position from frame to frame, a degree of "viscosity" is applied to it, using one of the many available known methods, such as a spline-based model. In brief, each frame is first processed independently and a first choice of sample volume position determined for each frame. Then the positions of all these sample volumes from all the frames are modelled by a function such as a spline or other noise-minimisation function. The purpose of this is to replace all these original sample volume positions with a new series of sample volume positions (one for each frame) but as though an elastic structure was passing through the images in a stack, and the elastic structure was reluctant to deviate drastically from one frame to the next. This helps prevent frames from succumbing to noise that causes a medically non-preferred sample volume to appear to the algorithm to be preferred, since a dramatic deviation from the other frame's preferred sample volumes would not be "followed" by the spline. A variety of methods for applying this "viscosity", or resistance to movement, of the sample volume between frames, are known to those skilled in the art.

Once the measurement positions at the annulae have been identified, the device can make peak velocity measurements at these sites using one of the following methods:

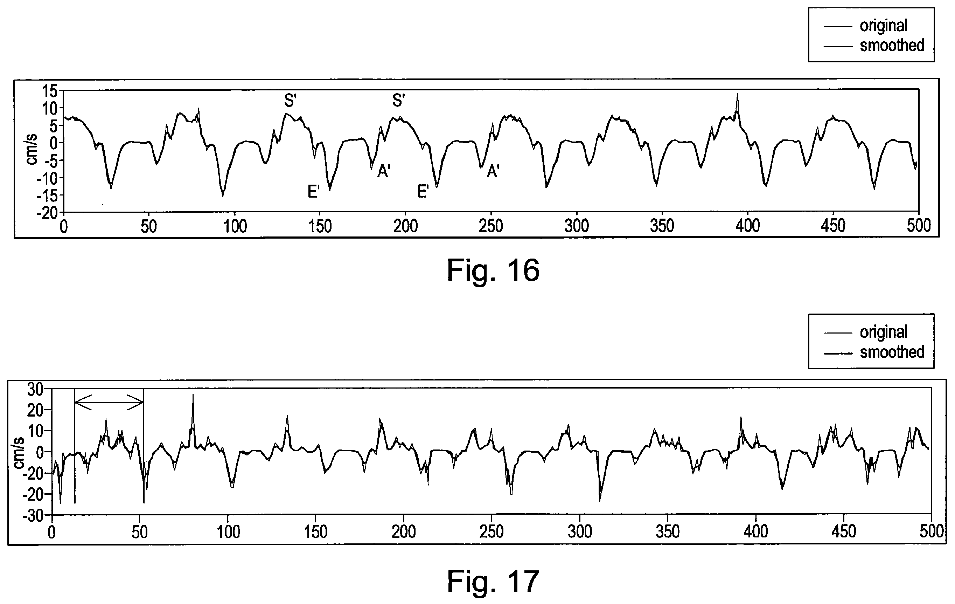

- Preferably, the device uses the speckle-tracked velocities at these positions, with peak detection methods to extract peak S', E' and A' velocities, which can be averaged across multiple beats. Figure 16 shows a speckle tracking velocity curve for an automatically detected annual septum.

- Alternatively the device can switch automatically to PW Doppler mode, placing the sample volume position at the measurement position selected by speckle tracking, and automatically analyse the Doppler traces obtained at these positions (see below) to measure the peak S', E' and A' velocities.

Conveniently, the ECG-free cardiac timing method described above can also be used to identify and eliminate corrupt or noisy beats during the real-time analysis. Using peak detection methods, the phases of the cardiac cycle can be identified and the cardiac timing curve can be split up into individual beats. In a particular embodiment, each beat should consist of both a systolic and diastolic phase. Beats with only a systolic phase or only a diastolic phase are assumed to be noisy e.g. due to movement of the probe, and are automatically classified as noisy beats. Those segments of the acquisition are excluded from further analysis. Figure 17 shows a speckle tracking velocity curve for the left wall showing a typical noisy beat to be removed from the sequence of beats.

The speckle tracked velocities at the annulae are measured using speckle tracking as described in the previous section. Since the measurements are made in real time, the device can make measurements averaged across multiple beats. Advantageously, this enables the device to provide error bars indicating the confidence in the measurement. A particular embodiment uses the cardiac timing information to identify individual beats from the cardiac timing curve, and overlay them to obtain an 'average' beat, with error bars showing the standard deviation.

The average velocity, $V_i$, is calculated as the mean of the velocity, $v_{ib}$, at position i for beat b, over a total number of n beats. Conveniently, this helps to quantify the quality of the acquired beats: good quality beats will reduce the error bars, whereas noisy beats will have very large error bars.

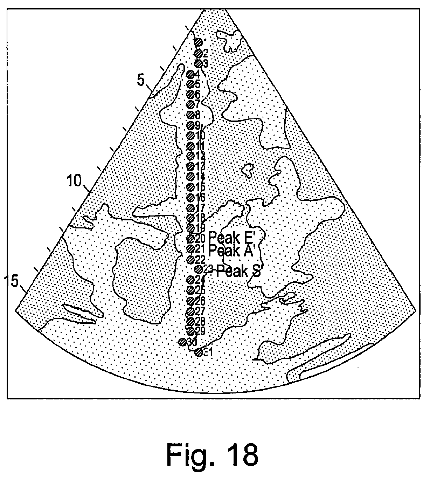

The current clinical method of measuring peak velocities, requires an expert echocardiographer to manually place a cursor at the crux of the heart, because in theory it is assumed that the peak systolic (S'), early diastolic (E') and late diastolic (A') myocardial velocities all occur at the same point in space, and it is impossible for operators to individually identify the exact location of the S', E' and A' peaks. The automated device according to the invention has velocity information along the septum from speckle-tracked velocities and can therefore provide the option to individually identify the peak velocities at each position during each phase of the cardiac cycle - systole, early diastole and late diastole. Using these velocities the algorithm can identify the specific positions along the septum at which each of the peak velocities (S', E' and A') should be measured. Figure 18 shows a greyscale apical four chamber freeze frame showing the positions of peak systolic (S'), early diastolic (E') and late diastolic (A') velocities, with labelled positions starting from 1 at the apex. The light numbered dots indicate the positions along the septum at which the velocities are measured. In the particular case shown in this figure, Peak E', A', and S' correspond to dots number 20, 21, and 23 respectively.

As described above, the mitral annulae can be located using the speckle-tracked velocity vectors. Since the valve inflow must lie between the two annulae this narrows down the region within which the inflow sample volume position should be placed. Therefore, the system only needs to search within this region. Additionally, the mitral valve position located using the anti-phase behaviour of the heart further reduces the search region to the localised region around the valve.

Preferably the device should then automatically switch to colour Doppler mode and reduce the colour flow box to the region around the valve (to maximise the frame rate), to precisely locate the sample volume position and make measurements. To this end, the sample volume position is sought as the point with the maximum upward velocity (peak diastolic velocity).

Less preferably, the speckle-tracked vectors in the region of the mitral valve can be used to locate the mitral valve leaflet tip, where the mitral inflow sample volume position should be placed.

Once the sample volume position has been selected, the device can make measurements of the peak early and late diastolic velocities by one of the following methods: preferably, switching into PW Doppler mode to acquire the transmitral Doppler trace and automatically analyse the trace using methods described below; less preferably, without switching to PW Doppler mode, examining the velocities from the 2D blood Doppler signal at the sample volume position. Because the frame rate of the 2D blood Doppler signal is low, it is preferable to (a) automatically zoom in the scan area to the region of interest (increasing the frame rate) and (b) automatically plot a parabola of velocity against time, during the time of blood movement, and from that parabola calculate the peak velocity by standard known formulae for quadratic equations.

If the device switches to PW Doppler mode to make peak velocity measurements, the trace acquired is automatically analysed to make velocity and displacement measurements. There are a number of known methods for PW spectrum analysis: Cloutier G, Allard L, Guo Z, Durand LG, "The effect of averaging cardiac Doppler spectrograms on the reduction of their amplitude variability", Med Biol Eng Comput. 1992 Mar;30(2):177-86; Tschirren J, Lauer RM, Sonka M, "Automated analysis of Doppler ultrasound velocity flow diagrams", IEEE Trans Med Imaging. 2001 Dec;20(12):1422-5; Lui EY, Steinman AH, Cobbold RS, Johnston KW, "Human factors as a source of error in peak Doppler velocity measurement", J Vasc Surg. 2005 Nov;42(5):972-9; Kiruthika, N.V. Prabhakar, B, Reddy, M.R. "Automated Assessment of Aortic Regurgitation using 2D Doppler Echocardiogram", Imagining Systems and Techniques, 2006. IST 2006. Proceedings of the 2006 IEEE International Workshop on Imagining read Imaging; Hirsch A, Petersch B, Hönigmann D, "Automated Doppler gate placement and velocity calculation based on a vessel angle estimate", Conf Proc IEEE Eng Med Biol Soc. 2006;1:4461-4; Guo, F, Park, JH, Carneiro G, Jackson J, Brendel M, Simopoulos C, Otsuki J, Comaniciu, D, "A probabilistic, hierarchical, and discriminant framework for rapid and accurate detection of deformable anatomic structure", 2007 IEEE 11th International Conference on Computer Vision; Magagnin V, Delfino L, Cerutti S, Turiel M, Caiani EG, "Nearly automated analysis of coronary Doppler flow velocity from transthoracic ultrasound images: validation with manual tracings", Med Biol Eng Comput. 2007 May;45(5):483-93; Park J, Zhou SK, Jackson J, Comaniciu D, "Automatic mitral valve inflow measurements from Doppler echocardiography", Med Image Comput Comput Assist Interv. 2008;11(Pt 1):983-90; Gaillard E, Kadem L, Clavel MA, Pibarot P, Durand LG, "Optimization of Doppler echocardiographic velocity measurements using an automatic contour detection method", Ultrasound Med Biol. 2010 Sep;36(9):1513-24; Turaga P, Beymer D, Wang, F, Amir A, Greenspan H, Pohl K, "Shape-based similarity retrieval of Doppler images for clinical decision support", 2010 IEEE Conference on Computer Vision and Pattern Recognition (CVPR); Nevo ST, et al, "Automated Tracking of the Mitral Valve Annulus Motion in Apical Echocardiographic Images Using Multidimensional Dynamic Programming", Ultrasound in Medicine & Biology. 2007;33(9):1389-99; Khan S et al, "Which echocardiographic Doppler left ventricular diastolic function measurements are most feasible in the clinical echocardiographic laboratory?", The American Journal of Cardiology. 2004;94(8):1099-101; Vinereanu D et al, "Reproducibility of pulsed wave tissue Doppler echocardiography. Journal of American Society of Echocardiography", 1999;12(6):492-9. Epub 1999/06/09; Gaillard E et al, "Optimization of Doppler velocity echocardiographic measurements using an automatic contour detection method", Annual International Conference of the IEEE Engineering in Medicine and Biology Society", 3-6 Sept. 2009; Greenspan H et al, "Doppler echocardiography flow-velocity image analysis for patients with atrial fibrillation", Ultrasound in Medicine & Biology. 2005;31(8):1031-40.

In a particular embodiment, the PW Doppler spectrum is traced along the middle of the envelope by searching for the highest intensity along each column in the image which represents the dominant velocity. Similarly the top and bottom extremes of the envelope, representing highest and lowest velocities respectively, are also traced, by moving outwards from the highest intensity point to the edge of the envelope. The three resulting traces (top, middle and bottom) are filtered using one of the many known available filters, preferably a Savitzky-Golay filter, to smooth out spikes due to noise while minimising unwanted disruption of the magnitude and timing of true underlying peaks and troughs. A peak detection algorithm is then applied to make measurements of peak velocities from the middle of the envelope and the top and bottom. For each peak, the velocity time integral (VTI) is also calculated by measuring the area under the curve. The device can compute an average peak/VTI measurement of multiple beats, with error bars. The error bars can be used in a similar method to those obtained from speckle tracking, to indicate to the operator the confidence in the measurements made.

An optional functionality of the device is automatic M-mode image analysis using a correlation based tracking algorithm. The algorithm starts by tracking all possible lines in the image using block matching starting at the extreme left of the image. Each block is compared to its neighbouring blocks to the right to find the direction of progression of the M-mode trace. Once all possible lines have been traced, the algorithm automatically identifies which lines are most likely to be valid based on the following criteria:

- More than a specified percentage, preferably 50%, of the line should have an underlying contrast (this helps to eliminate lines which disappear along the M-mode trace);

- The average excursion (positive and negative) of the line should be close to zero (this helps to eliminate lines trailing off);

- The average absolute excursion should be greater than zero (this helps to eliminate straight lines across the M-mode which do not represent tissue motion).

The lines which meet the above criteria are then correlated with each other, to identify those that are moving in the same pattern of motion since tissues ideally should be moving in relatively the same pattern. The lines are sorted in order of correlation coefficient, and only the top specified percentage, preferably 30%, of the lines are used as valid lines.

Once the valid lines have been selected, the algorithm averages across these lines to obtain an overall trace. This trace is filtered by one of many known filters, and is used to obtain measurements of excursion by finding the peaks and troughs. Preferably, the filter is a Savitzky-Golay filter. The trace is then differentiated to obtain a velocity-time trace (which is very similar to the pattern of a Doppler trace curve). This curve can be used to make automatic measurements of peak S', E' and A' velocities.

If the embodiment includes simultaneous acquisition of an electrocardiogram signal, the system can also provide timing information relative to the ECG, i.e. time from R-wave to peak S'.

Experimentation has shown that, in addition to beat-to-beat variability, an important contributor to variability in measurements of cardiac performance and circulating volume status is probe position. To obtain the most accurate result, measurements should be obtained not only from several beats, but from different probe positions. In an embodiment of the present invention, a motion sensor within the probe guidance system detects when the probe has been held in the same position for a certain period of time. The system will then stop recording data and indicate to the operator to remove the probe from the chest and then replace it on the chest. When the motion sensor has detected the period of movement associated with repositioning of the probe has ended, the system restarts the timer as another period of stability is detected during which measurements are made.

Effective regurgitant orifice area (EROA) in mitral regurgitation (MR) is difficult to quantify. Clinically it is measured using the proximal iso-velocity surface area (PISA) method, which is intrinsically not automatable, because it requires the operator to manually identify the mitral valve orifice. The fully automated algorithm, ("AQURO"), calculates EROA directly from echocardiographic colour M-mode data, without requiring operator input.

By eliminating the need to identify the orifice location, AQURO avoids an important source of measurement variability. Compared with PISA, it also reduces the analysis time allowing analysis and averaging of data from significantly more beats, improving the consistency of EROA quantification. AQURO, being fully automated, is a simple, effective enhancement for EROA quantification using standard echocardiographic equipment. A full description is provided by Moraldo M, Bergamini C, Malaweera AS, Dhutia NM, Pabari PA, Willson K, Baruah R, Manisty C, Davies JE, Xu XY, Hughes AD, Francis DP, "A novel fully automated method for mitral regurgitant orifice area quantification", Int J Cardiol. 2012.

The disclosed system presents the information to operators using a number of novel strategies to ensure that the significance of results is appreciated by front-line healthcare personnel without specialist training.

Normal measurements of the heart vary with age. Incorporating the patient's age reduces the risk of misdiagnosis (Chahal NS, Lim TK, Jain P, Chambers JC, Kooner JS, Senior R, "Normative reference values for the tissue Doppler imaging parameters of left ventricular function: a population-based study", Eur J Echocardiogr 2010;11(1):51-56), but is currently rarely undertaken even by experts because of the time involved. The disclosed system present a Z-score, rather than the raw measurement, so that it is immediately clear to the operator when a result is abnormal having taken into account the patient's age. A z-score indicates the number of standard deviations that the measurement varies from the mean measurement for the expected statistical distribution of measurements for the patient age.

By making several automated measurements, the disclosed system is able to provide confidence intervals for the Z-score presented, so that clinicians can appreciate how likely it is the result is genuinely abnormal, or whether it is a lower-quality measurement that should be interpreted with caution.

By recording and analysing measurements of cardiac performance and circulating volume status in real-time, the disclosed system is able to decide how many beats are required before reporting a Z-score to the operator. In situations where there is widespread variability in measurements, the disclosed system detects this and measures more beats before presenting a result to the operator. In situations where the result is far from expected, more beats will be scrutinised to verify that the result is genuine.

The disclosed system presents the measurement not only as a Z-score but as a graphical representation of a normal distribution, with colour-coding of hypothetical patients up to the 5th centile, and a marker indicating where on the distribution the patient's measurement lies.

In summary, various methods for automatically processing an ultrasound image for echocardiography are disclosed in which the regions of interest in the ultrasound image are identified by determining velocity vectors for tissue movement in the image. A novel construction of ultrasound probe is also disclosed.

Throughout the description and claims of this specification, the words "comprise" and "contain" and variations of them mean "including but not limited to", and they are not intended to (and do not) exclude other components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The work leading to this invention has received funding from the European Research Council under the European Union's Seventh Framework Programme (FP7/2007- 2013) / ERC grant agreement n° 281524.

**Claims**

1. A method for automatically processing an ultrasound image for echocardiography, the method comprising:

   determining an average velocity vector for tissue movement in each frame of the image;
   monitoring the change in at least a component of the average velocity vector between subsequent frames to identify at least one of a minimum and a maximum of the (component of the) average velocity vector;
   and on the basis of the identified maximum or minimum providing cardiac timing information.

2. A method as claimed in claim 1, wherein the component of the average velocity vector is a component in the direction of the apex of the heart in the image.

3. A method as claimed in claim 1 or 2, wherein the monitoring step comprises at least one of identifying a minimum corresponding to the early diastolic phase of the cardiac cycle, identifying a minimum corresponding to the late diastolic phase of the cardiac cycle and identifying a maximum corresponding to the systolic phase of the cardiac cycle.

4. A method for automatically processing an ultrasound image for echocardiography, the method comprising:

   determining velocity vectors for tissue movement in the image;
   assigning confidence values to the determined velocity vectors;
   examining a section of the image in a direction substantially transverse to the apical direction of the image;
   identifying three regions within the examined section having the highest velocities and confidence values;
   providing an indication that the three regions correspond to the medial wall, septum and lateral wall of the heart, respectively.

5. A method as claimed in claim 4 further comprising automatically locating the mitral or triscuspid valve by identifying at least a first region of the image in which the average velocity vector is in the direction of the apex of the heart during the diastolic phase of the cardiac cycle; and providing an indication that the first region corresponds to the mitral or tricuspid valve and automatically locating the medial annulus, septal annulus or lateral annulus by reference to the location of the valve and the location of the medial wall, septum and lateral wall.

6. A method as claimed in claim, wherein the velocity vectors are determined by block matching pixels in sequential frames of the ultrasound image.

7. A method as claimed in claim 6 comprising assigning a confidence value to each determined

8. A method as claimed in claim 7, wherein the confidence value is determined on the basis of at least one of: contrast of the pixels in the matched block; distribution of the values of the pixels in the matched block relative to neighbouring blocks; correlation between the variation of at least a component of the determined velocity vector and (the corresponding component) of the average velocity vector for the image.

9. A method as claimed in claim 7 or 8 comprising identifying a plurality of regions within the image having confidence values above a minimum value.

10. A method as claimed in any of claims 6 to 9 comprising calculating an average velocity vector for each region.

11. A method as claimed in claim 10 comprising calculating an average velocity vector for tissue movement in the image as the average of the average velocity vectors for each region.

12. Computer software which configures data processing apparatus to automatically process an ultrasound image for echocardiography in accordance with the method of any preceding claim.

13. Data processing apparatus configured to automatically process an ultrasound image for echocardiography in accordance with the method of any of claims 1 to 12.

14. An ultrasound imaging system comprising data processing apparatus as claimed in claim 13 and an ultrasound probe.

15. An ultrasound imaging system as claimed in claim 14, wherein the system is a real-time ultrasound imaging system.

Fig. 1

Probe

Fig. 2

Fig. 3

Fig. 4

## Fig. 5

## Fig. 6

| Unsatisfactory-Move Probe to Left | Satisfactory-Measurements Acquired | Unsatisfactory-Move Probe to Right |
|---|---|---|

Operator Corrects Probe Position

Operator Corrects Probe Position

## Fig. 7

Initial kernel

Search window

A

B

## Fig. 8

Best-matched grid

Velocity vector

Initial grid

Search window

## Fig. 9

Medially translated
probe

On-axis

Laterally translated
probe

## Fig. 10

Medially tilted
probe

On-axis

Laterally tilted
probe

## Fig. 11

Fig. 12

Fig. 13

Fig. 14

Fig. 15

Fig. 16

Fig. 17

31

Fig. 18

**EP 3 056 153 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **L.N. BOHS ; G.E. TRAHEY.** A novel method for angle independent ultrasonic imaging of blood flow and tissue motion. *IEEE Trans Biomed Eng,* 1991, vol. 38, 280-286
- **L.N. BOHS ; B. GEIMAN ; M. ANDERSON ; S. GEBHART ; G.E. TRAHEY.** Speckle tracking for multidimensional flow estimation. *Ultrasonics,* 2000, vol. 38, 369-375
- **GOFFINET, C. ; VANOVERSCHELDE, JL.** Speckle Tracking echocardiography. *In: European Cardiovascular Disease,* 2007
- **F. YEUNG ; S.F. LEVINSON ; K.J. PARKER.** Multilevel and motion model-based ultrasonic speckle tracking algorithm. *Ultrasound Med Biol,* 1998, vol. 24 (3), 427-441
- A comparison of algorithms for tracking sub-pixel speckle motion. **B. GEIMAN ; L. BOHS ; M. ANDERSON ; S. BREIT ; G. TRAHEY.** Ultrasonics Symp. Volume. IEEE, 1997, vol. 2, 1239-1242
- Methods for the estimation of sub-sample motion of digitized ultrasound echo signals in two dimensions. **ZAHIRI-AZAR, R. ; GOKSEL, O. ; YAO, T.S. ; DEHGHAN, E. ; YAN, J. ; SALCUDEAN, S.E.** EMBS. 2008
- *30th Annual International Conference of the IEEE,* 2008, 5581-5584
- **MICHELSON, A.** Studies in Optics. U. of Chicago Press, 1927
- **THOMAS H. MARWICK ; CHEUK-MAN YU ; JING PING SUN.** Myocardial Imaging: Tissue Doppler and Speckle Tracking. John Wiley & Sons, 2008
- **WANG Z. A.** Universal Image Quality Index. *IEEE Signal Processing Letters,* 2002, vol. 9 (3), 81-4
- **HUITAO L.** A training-based no-reference image quality assessment algorithm. *International Conference on Image Processing, ICIP,* 2004, vol. 04
- **XIN L.** Blind Image Quality Assessment. *IEEE International Conference on Image Processing,* 2002, vol. 1, 449-52
- **AASE SA ; SNARE SR ; DALEN H ; STØYLEN A ; ORDERUD F ; TORP H.** Echocardiography without electrocardiogram. *Eur J Echocardiogr,* 2011, vol. 12, 3-10
- **GIFANI P ; BEHNAM H ; SHALBAF A ; SANI ZA.** Automatic detection of end-diastole and end-systole from echocardiography images using manifold learning. *Physiol Meas,* 2010, vol. 31, 1091-103

- Doppler and transmitral Doppler measurements of E, E' and E/E. *Int J Cardiol.,* vol. 155 (3), 394-9
- **CLOUTIER G ; ALLARD L ; GUO Z ; DURAND LG.** The effect of averaging cardiac Doppler spectrograms on the reduction of their amplitude variability. *Med Biol Eng Comput.,* March 1992, vol. 30 (2), 177-86
- **TSCHIRREN J ; LAUER RM ; SONKA M.** Automated analysis of Doppler ultrasound velocity flow diagrams. *IEEE Trans Med Imaging,* December 2001, vol. 20 (12), 1422-5
- **LUI EY ; STEINMAN AH ; COBBOLD RS ; JOHNSTON KW.** Human factors as a source of error in peak Doppler velocity measurement. *J Vasc Surg,* November 2005, vol. 42 (5), 972-9
- **KIRUTHIKA, N.V. ; PRABHAKAR, B ; REDDY, M.R.** Automated Assessment of Aortic Regurgitation using 2D Doppler Echocardiogram. *Imagining Systems and Techniques,* 2006
- **HIRSCH A ; PETERSCH B ; HÖNIGMANN D.** Automated Doppler gate placement and velocity calculation based on a vessel angle estimate. *Conf Proc IEEE Eng Med Biol Soc.,* 2006, vol. 1, 4461-4
- **GUO, F ; PARK, JH ; CARNEIRO G ; JACKSON J ; BRENDEL M ; SIMOPOULOS C ; OTSUKI J ; COMANICIU, D.** A probabilistic, hierarchical, and discriminant framework for rapid and accurate detection of deformable anatomic structure. *IEEE 11th International Conference on Computer Vision,* 2007
- **MAGAGNIN V ; DELFINO L ; CERUTTI S ; TURIEL M ; CAIANI EG.** Nearly automated analysis of coronary Doppler flow velocity from transthoracic ultrasound images: validation with manual tracings. *Med Biol Eng Comput.,* May 2007, vol. 45 (5), 483-93
- **PARK J ; ZHOU SK ; JACKSON J ; COMANICIU D.** Automatic mitral valve inflow measurements from Doppler echocardiography. *Med Image Comput Comput Assist Interv.,* 2008, vol. 11, 983-90
- **GAILLARD E ; KADEM L ; CLAVEL MA ; PIBAROT P ; DURAND LG.** Optimization of Doppler echocardiographic velocity measurements using an automatic contour detection method. *Ultrasound Med Biol.,* September 2010, vol. 36 (9), 1513-24
- **TURAGA P ; BEYMER D ; WANG, F ; AMIR A ; GREENSPAN H ; POHL K.** Shape-based similarity retrieval of Doppler images for clinical decision support. *IEEE Conference on Computer Vision and Pattern Recognition (CVPR),* 2010

33

- **NEVO ST et al.** Automated Tracking of the Mitral Valve Annulus Motion in Apical Echocardiographic Images Using Multidimensional Dynamic Programming. *Ultrasound in Medicine & Biology,* 2007, vol. 33 (9), 1389-99
- **KHAN S et al.** Which echocardiographic Doppler left ventricular diastolic function measurements are most feasible in the clinical echocardiographic laboratory?. *The American Journal of Cardiology,* 2004, vol. 94 (8), 1099-101
- **VINEREANU D et al.** Reproducibility of pulsed wave tissue Doppler echocardiography. *Journal of American Society of Echocardiography,* 1999, vol. 12 (6), 492-9
- **GAILLARD E et al.** Optimization of Doppler velocity echocardiographic measurements using an automatic contour detection method. *Annual International Conference of the IEEE Engineering in Medicine and Biology Society,* 03 September 2009
- **GREENSPAN H et al.** Doppler echocardiography flow-velocity image analysis for patients with atrial fibrillation. *Ultrasound in Medicine & Biology,* 2005, vol. 31 (8), 1031-40
- **MORALDO M ; BERGAMINI C ; MALAWEERA AS ; DHUTIA NM ; PABARI PA ; WILLSON K ; BARUAH R ; MANISTY C ; DAVIES JE ; XU XY.** A novel fully automated method for mitral regurgitant orifice area quantification. *Int J Cardiol.,* 2012
- **CHAHAL NS ; LIM TK ; JAIN P ; CHAMBERS JC ; KOONER JS ; SENIOR R.** Normative reference values for the tissue Doppler imaging parameters of left ventricular function: a population-based study. *Eur J Echocardiogr,* 2010, vol. 11 (1), 51-56